# EUROPEAN PATENT APPLICATION

(11) **EP 2 062 612 A1**
(43) Date of publication of application: **27.05.2009**
(21) Application number: 07792661.6
(22) Date of filing: 17.08.2007
(51) Int. Cl.: A61M 37/00

(54) **MICRONEEDLE AND MICRONEEDLE PATCH**

(30) Priority: 18.08.2006 JP 2006223601
(71) Applicant: Toppan Printing Co., Ltd., Taito-ku, Tokyo 110-0016 (JP)
(72) Inventor: TOMONO, Takao, 5-1, Taito 1-chome, Taito-ku, Tokyo 110-0016 (JP)
(74) Representative: TBK-Patent
(86) International application number: PCT/JP2007/066045
(87) International publication number: WO 2008/020633

(57) **Abstract**

Insertion of a micro-needle into a living body is made easy. The micro-needle (121) includes first and second end sections (121a, 121b) arranged in a longitudinal direction and includes a biocompatible material, wherein the first end section (121a) tapers down from it's end on a side of the second end section (121b) toward another end thereof and the maximum apical angle of the first end section (121a) falls within a range of 9 to 53°.

## Description

### Technical Field

The present invention relates to a micro-needle patch applied, for example, to a surface of a living body.

### Background Art

Generally, transdermal administration of a drug to a living body includes application of a liquid or viscous body containing the drug to the skin. However, the applied drug is prone to be removed from the surface of the skin due to perspiration or contact. In addition, when the applied drug is intended to penetrate into the inner layer of the skin, the degree of penetration is difficult to control.

In this connection, use of a micro-needle array for administration of a drug is proposed. A micro-needle array has a structure in which micro-needles are arranged on a substrate. For example, JP-A 2003-238347 (KOKAI) describes a micro-needle array including a polymethylmethacrylate substrate and micro-needles of maltose formed thereon.

For administration of a drug with a micro-needle array, used is a micro-needle array whose micro-needles contain the drug, for example. To be more specific, such a micro-needle array is pressed against the skin to insert the micro-needles into the living body. In the case where the micro-needles contain a drug, by leaving the micro-needles in the living body, it is possible to prevent the drug from being removed from the living body due to perspiration, contact, etc. In addition, the degree of penetration of the drug can be controlled, for example, according to the lengths and/or density of the micro-needles.

A micro-needle array is required that the micro-needles are inserted into the living body with reliability. However, the present inventor has found out the following fact in the course of animal tests in achieving the present invention. That is, most of the micro-needles, for example, the micro-needles that contain maltose as a main component are difficult to insert into the living body.

### Disclosure of Invention

An object of the present invention is to provide a micro-needle that is easy to be inserted into the living body.

According to a first aspect of the present invention, there is provided a micro-needle comprising first and second end sections arranged in a longitudinal direction and including a biocompatible material, the first end section tapering down from an end of the first end section on a side of the second end section toward another end of the first end section, a minimum dimension of the first end section in a width direction perpendicular to the longitudinal direction being smaller than a minimum dimension of the second end section in the width direction, a maximum apical angle of the first end section falling within a range of 9 to 53°, the maximum apical angle being a maximum of apical angles each defined as an angle that a first straight line passing through first and second intersection points forms with a second straight line passing through third and fourth intersection points, the first and third intersection points being intersection points of a first plane and a contour of an orthogonal projection of the micro-needle on a projection plane parallel with the longitudinal direction, the second and fourth intersection points being intersection points of a second plane and the contour, the first plane being perpendicular to the longitudinal direction and spaced apart from the another end by one tenth of a length of the micro-needle in the longitudinal direction, and the second plane being perpendicular to the longitudinal direction and spaced apart from the another end by one third of the length.

According to a second aspect of the present invention, there is provided a micro-needle patch, comprising a support layer with first and second main surfaces, and micro-needles each extending from the first main surface, each of the micro-needles being the micro-needle according to the first aspect supported by the first main surface at an end of the second end section.

### Brief Description of Drawings

FIG. 1 is a perspective view schematically showing a micro-needle patch according to an embodiment of the present invention;
FIG. 2 is a perspective view schematically showing the micro-needle patch shown in FIG. 1 provided with a protection member;
FIG. 3 is a perspective view schematically showing a part of the micro-needle patch shown in FIG. 1;
FIG. 4 is a perspective view schematically showing a micro-needle included in the structure shown in FIG. 3;
FIG. 5 is a perspective view schematically showing an example of modified micro-needle;
FIG. 6 is a perspective view schematically showing an example of modified micro-needle;
FIG. 7 is a perspective view schematically showing an example of modified micro-needle;
FIG. 8 is a perspective view schematically showing an example of modified micro-needle;
FIG. 9 is a perspective view schematically showing an example of modified micro-needle;
FIG. 10 is a perspective view schematically showing an example of modified micro-needle;
FIG. 11 is a perspective view schematically showing an example of modified micro-needle;
FIG. 12 is a perspective view schematically showing an example of modified micro-needle;
FIG. 13 is a perspective view schematically showing an example of modified micro-needle;
FIG. 14 is a perspective view schematically showing a still another example of modified micro-needle;
FIG. 15 is a view showing an orthogonal projection of the micro-needle shown in FIG. 14 onto a plane perpendicular to the longitudinal direction thereof;
FIG. 16 is a flow-chart showing an example of a method of manufacturing a micro-needle patch;
FIG. 17 is a view schematically showing a part of a tension and compression-testing machine;
FIG. 18 is a graph showing relationships between an apical angle and a puncturing performance and a resistance to breaking of a micro-needle;
FIG. 19 is a sectional view schematically showing a structure of a micro-needle employed in Example 2;
FIG. 20 is a sectional view schematically showing a structure of a micro-needle employed in Example 2;
FIG. 21 is a sectional view schematically showing a structure of a micro-needle employed in Example 2; and
FIG. 22 is a sectional view schematically showing a structure of a micro-needle employed in Example 2.

### Best Mode for Carrying Out the Invention

An embodiment of the present invention will be described below. In the drawings, the same reference symbols denote components having the same or similar functions and duplicate descriptions will be omitted.

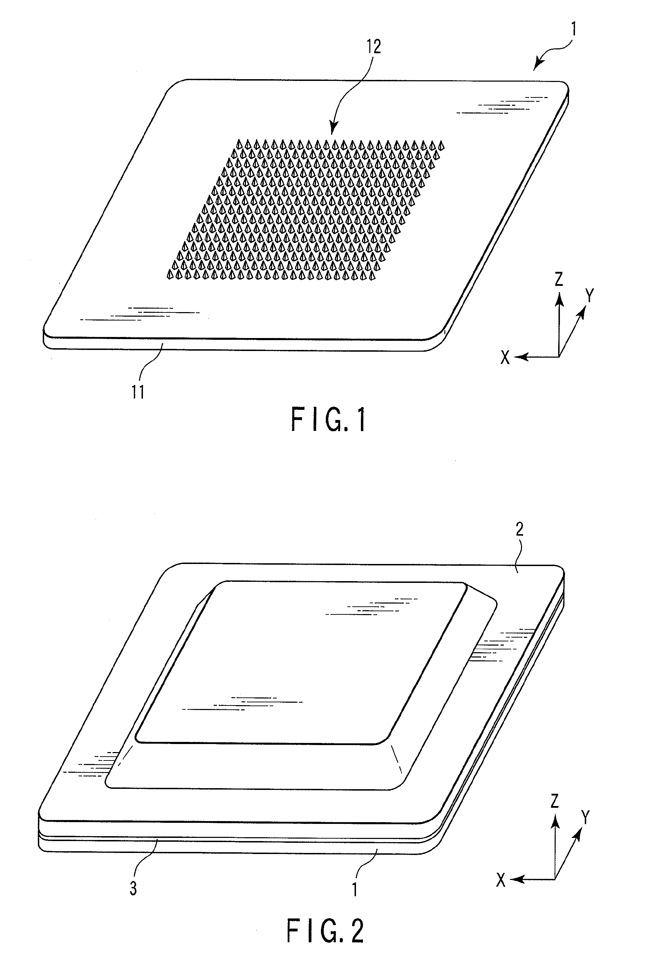

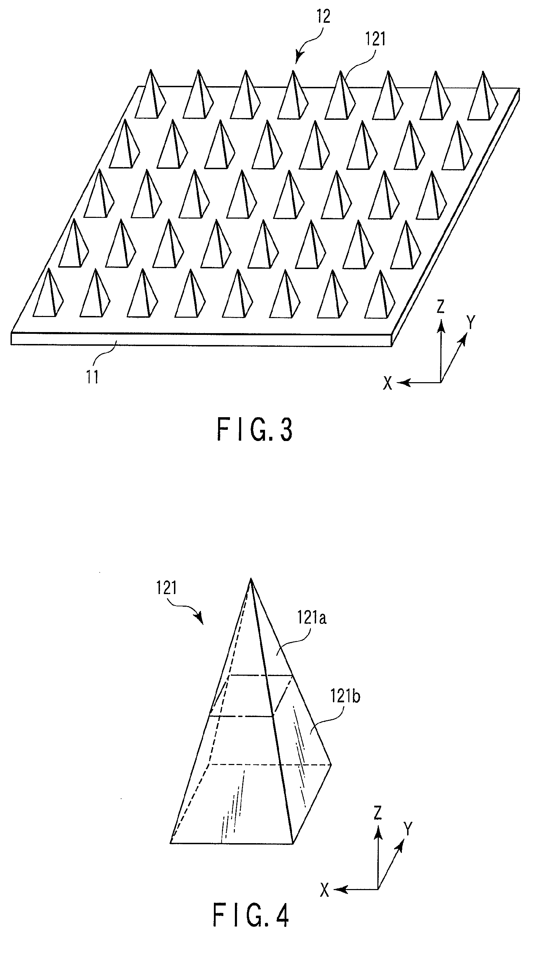

FIG. 1 is a perspective view schematically showing a micro-needle patch according to an embodiment of the present invention. FIG. 2 is a perspective view schematically showing the micro-needle patch shown in FIG. 1 provided with a protection member. FIG. 3 is a perspective view schematically showing a part of the micro-needle patch shown in FIG. 1. FIG. 4 is a perspective view schematically showing a micro-needle included in the structure shown in FIG. 3.

Note that in FIGS. 1 to 4, the X and Y directions are the directions parallel with a main surface of the micro-needle patch and perpendicular to each other. Note also that the Z direction is the direction perpendicular to the X and Y directions.

The micro-needle patch 1 shown in FIG. 1 includes a support layer 11 and a micro-needle array 12. The support layer 11 includes first and second main surfaces. The first main surface supports the micro-needle array 12.

Before using the micro-needle patch 1, the micro-needle array 12 is protected, for example, using the protection member 2 shown in FIG. 2. The protection member 2 shown in FIG. 2 is a plate-like molded article recessed at the position corresponding to the micro-needle array 12, and adhered to the support layer 11 via the adhesive layer 3. When the micro-needle patch 1 is used, it is removed from the protection member 2. Then, the micro-needle patch 1 is pressed against a living body such that the micro-needle array 12 is inserted therein.

Next, the constituents of the micro-needle patch 1 will be described in more detail.

The support member 11 shown in FIGS. 1 and 3 has a monolayer structure or multilayered structure. The support layer 11 may be rigid or flexible. As the material of the support layer 11, for example, organic polymer such as plastic, metal, glass or a mixture thereof may be used. When a multilayered structure is employed in the support layer 11, a part thereof may be a cloth or paper. Typically, the main surface of the support layer 11 on the side of the micro-needle array 12 is made of the same or almost the same material as that of the micro-needle array 12.

As shown in FIG. 3, the micro-needle array 12 is composed of micro-needles 121. The micro-needles 121 extend from the first main surface of the support layer 11.

As shown in FIG. 4, each micro-needle 121 includes a first end section 121a and a second end section 121b arranged in a longitudinal direction. Note that in FIG. 4, the plane drawn in the alternate long and short dash line shows the boundary surface between the first end section 121a and the second end section 121b.

The first end section 121a tapers down from an end on the side of the second end section 121b toward another end so that it is easily inserted into a living body. On the other hand, the minimum dimension of the second end section 121b in a width direction perpendicular to the longitudinal direction is greater than that of the first end section 121a so that the support layer 11 can hold the micro-needle 121 at a sufficient strength.

To be more specific, the first end section 121a has roughly a quadrangular pyramid shape. The second end section 121b has roughly a truncated quadrangular pyramid shape. The first end section 121a and the second end section 121b are equal in angles of inclinations of lateral faces. In addition, the lateral faces of the first end section 121a are flush with the lateral faces of the second end section 121b. That is, each micro-needle 121 has roughly a quadrangular pyramid shape whose base is parallel with the X and Y directions.

Further, the base of the micro-needle 121 includes a pair of edges parallel with the X direction and a pair of edges parallel with the Y direction. The dimension of the first end section 121a in the Z direction is, for example, equal to or more than one third of the dimension of the micro-needle 121 in the Z direction.

In each micro-needle 121, the maximum apical angle of the first end section 121a falls within a range of 9 to 53°, and typically falls within a range of 20 to 30°. Also, in each micro-needle 121, the minimum apical angle falls, for example, within a range of 9 to 53°, and typically within a range of 20 to 30°. The "maximum apical angle" and the "minimum apical angle" will be defined later.

In the case where the apical angle of the first end section 121a is small, the micro-needles 121 prone to be broken when the micro-needle patch 1 is applied to a living body. In the case where the apical angle is large, a stronger force is necessary for inserting the micro-needles 121 into the surface of a living body as compared with the case where the apical angle is small. That is, in the case where the apical angle is large, it is difficult to smoothly insert the micro-needles 121 into the surface of a living body.

The dimension of the micro-needles 121 in the Z direction is, for example, within a range of about 20 µm to about 1.4 mm. As will be described below, the dimension can be determined according to the application of the micro-needle patch 1.

The skin of human has a three-layered structure of epidermis, dermis and subcutaneous tissue. The thickness of the epidermis is within a range of about 0.07 mm to about 0.2 mm. The thickness of the stratum corneum is about 0.02 mm. The thickness of the skin constituted by the epidermis and the dermis is within a range of about 1.5 mm to about 4 mm.

The feed substance such as the bioactive substance cannot penetrate into the body unless the substance reaches to the dermis. Thus, for such an application, the dimension of the micro-needles 121 in the Z direction is set, for example, at about 0.02 mm or more, and typically at about 0.2 mm or more. In order to insert the micro-needles 121 through the epidermis with reliability, the dimension of the micro-needles 121 in the Z direction is set, for example, at about 0.3 mm or more. In order to insert the micro-needles 121 through the skin with reliability, the dimension of the micro-needles 121 in the Z direction is set, for example, at about 4 mm or more.

The maximum dimension of the micro-needles 121 parallel with the XY plane is, for example, about 300 µm or less. The dimension can be determined, for example, in consideration of pain that the micro-needles 121 make the living body feel.

An injection needle having a thickness of 0.2 mm is commercially available as a painless needle. In order to make a human feel no pain, the maximum dimension of the micro-needles 121 parallel with the XY direction should be, for example, about 0.15 mm or less, and typically within a range of about 0.05 mm to about 0.07 mm.

The micro-needles 121 include a biocompatible material. Typically, the biocompatible material is a biocompatible and biodegradable material. In this case, as the biocompatible material, for example, a material having a half-life in a living body of about one month or less is used. As the biocompatible material, for example, chitin and/or chitosan, polylactic acid, a copolymer of polylactic acid and glycolic acid, magnesium compound or titanium compound shown in the table below can be used.

Note that chitosan is a deacetylated product of chitin. Note also that "chitin and/or chitosan" refers to at least one of chitin and chitosan, and typically is chitosan or a mixture of chitin and chitosan. Hereinafter, "chitin and/or chitosan" is abbreviated to "chitin/chitosan".

**TABLE 1**

| Main component of material | Young's modulus (GPa) | Tensile strength (MPa) | Decomposition rate (half-life) |
|---|---|---|---|
| Chitin/chitosan | 6 | 60 | 2 weeks |
| PLA | 1.5-2.5 | 20-60 | 1 month-1 year |
| PLGA | 2-9 | 40-850 | 10 weeks-7 months |
| Mg | 45 | 230 | 2-3 weeks |
| Ti | 110 | 320 | - |
| SUS304 (injection needle) | 197 | 520 | - |

In the table above, "PLA" denotes polylactic acid, "PLGA" denotes a copolymer of polylactic acid and glycolic acid, "Mg" denotes a magnesium compound, and "Ti" denotes a titanium compound. Note that the magnesium compound and the titanium compound are the compounds generally used for an artificial bone. Note also that the numerical values in the above table are only examples, and may slightly vary according molecular weight, etc.

Skin of a living body has elasticity. For example, epidermis, dermis and subcutaneous tissue of a human have Young's moduli of about 0.14 MPa, about 0.080 MPa and about 0.034 MPa, respectively.

In order to insert a needle into the epidermis, the force stronger than the Young's modulus of the epidermis is necessary. In order to insert the needle into the epidermis with reliability, the force should be over about 100 times, preferably over about 1,000 times the Young's modulus of the epidermis. On the other hand, in order to withdraw the needle, the tensile strength of the needle should be, for example, 5 MPa or more, desirably 50 MPa or more.

The biocompatible materials shown in the above table have a sufficient Young's modulus. Thus, the micro-needles 121 including the biocompatible materials can be easily inserted into a living body. Therefore, for example, when a predetermined amount of a feed substance is supported by surfaces of the micro-needles 121, the feed substance can be fed into the living body at almost the same amount as the design value.

In addition, the biocompatible materials shown in the above table have a sufficient tensile strength. Therefore, the micro-needles 121 including the biocompatible materials resist breaking when they are withdrawn from the living body.

Furthermore, in the case where a biocompatible material having biodegradable property is used, if a broken micro-needle 121 is left in a living body, the micro-needle 121 hardly prevents the healing of a wound caused by pressing the micro-needle patch 1 against a surface of the living body. In particular, chitin/chitosan has hemostatic and bactericidal properties. Therefore, the micro-needles 121 including chitin/chitosan accelerate the stopping up of the wound caused by pressing the micro-needle patch 1 against a surface of the living body so as to prevent the invasion of viruses into the living body, and inhibit the growth of viruses in the living body. That is, the micro-needle 121 left in the living body encourages the healing of the wound caused by pressing the micro-needle patch 1 against a surface of the living body.

As the feed substance described above, for example, a bioactive substance that acts on a structural element of a living body, a bioinert substance that does not act on a structural element of a living body, or a mixture thereof can be used. As the bioactive substance, one or more substances that can cause a physiological change in a living body when administered to the living body, for example, drugs. As this drug, for example, insulin, ketamine, nitroglycerin, isosorbide dinitrate, estradiol, tulobuterol, nicotine, scopolamine or clonidine hydrochloride can be used. As the bioinert substance, for example, one or more substances used in cosmetics such as dye and humectant can be used.

The biocompatible material content of the micro-needles 121 is set, for example, at 50% by mass or more. When the content is small, Young's modulus and/or tensile strength of the micro-needles 121 may be insufficient.

Various modifications to the micro-needles 121 can be possible.

In the micro-needle 121 shown in FIG. 4, the first end section 121a has roughly a quadrangular pyramid shape. The first end section 121a may have another shape. For example, the first end section 121a may be a cylinder such as circular cylinder, elliptic cylinder and prism. The cylinder may be a right cylindrical body, an oblique cylindrical body or a truncated cylindrical body. However, the first end section 121a typically employs the structure in which it is tapered down from an end on the side of the second end section 121b to another end. In this case, the first end section 121a may be, for example, a cone such as circular cone, elliptic cone and pyramid. The cone may be a right cone, an oblique cone, a right truncated cone or an oblique truncated cone.

In the micro-needle 121 shown in FIG. 4, the second end section 121b has roughly a truncated quadrangular pyramid shape. The second end section 121b may have another shape. For example, the second end section 121b may be a cylinder such as circular cylinder, elliptic cylinder and prism. Alternatively, the second end section 121b may be tapered down from an end on the side of the first end section 121a to another end. In this case, the second end section 121b may be, for example, a truncated cone such as circular truncated cone, elliptic truncated cone and truncated pyramid. The truncated cone may be a right truncated cone or an oblique truncated cone. However, the second end section 121b typically employs the structure in which it is tapered down from an end on the side of the support layer 11 to another end. In this case, the second end section 121b may be, for example, a truncated cone such as truncated circular cone, truncated elliptic cone and truncated pyramid. The truncated cone may be a right truncated cone or an oblique truncated cone.

In the micro-needle 121 shown in FIG. 4, the micro-needle 121 has roughly a quadrangular pyramid shape whose base is parallel with the X and Y directions. The micro-needle 121 may have another shape. For example, the micro-needle 121 may have any shape obtained by combining the shape described for the first end section 121a with the shape described for the second end section 121b. However, the micro-needle 121 typically employs the structure in which it is tapered down from an end of the support layer 11 to another end. In this case, the micro-needle 121 may be, for example, a cone such as circular cone, elliptic cone and pyramid. The cone may be a right cone, an oblique cone, a right truncated cone or an oblique truncated cone. Alternatively, the micro-needle 121 may have the shape obtained by combining the first end section 121a having a cone shape with the second end section 121b having a cylindrical shape.

At least one of the micro-needles 121 may have a symmetry axis parallel with the longitudinal direction thereof. Such a micro-needle 121 resists breaking when it is pressed against the surface of a living body.

At least one of the micro-needles 121 may be asymmetric. For example, at least one of the micro-needles 121 may have no symmetrical axis parallel with the longitudinal direction thereof. In this case, the micro-needle 121 is prone to be broken when applied with a force in a direction crossing the Z direction as compared with the case where the micro-needle 121 has a symmetrical axis parallel with the Z direction.

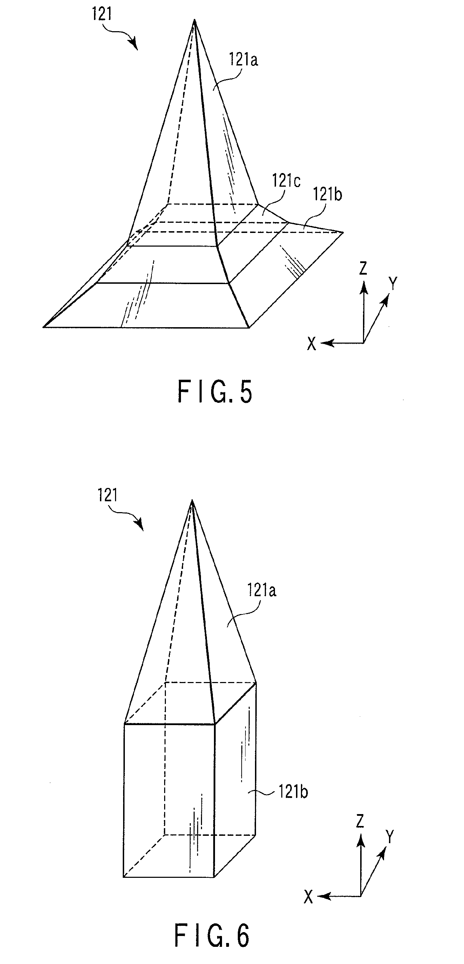

FIGS. 5 to 13 are perspective views schematically showing examples of modified micro-needle.

The micro-needle 121 shown in FIG. 4 has the structure in which it is tapered down from an end on the side of the support layer 11 to another end. The first end section 121a has a quadrangular pyramid shape. The second end section 121b has a truncated quadrangular pyramid shape. The angles that the lateral faces of the first end section 121a make with the Z direction are smaller than the angles that the lateral faces of the second end section 121b make with the Z direction.

As such, the first end section 121a and the second end section 121b may be different from each other in the angles of inclinations of lateral faces. When such a structure is employed in which the angles that the lateral faces of the first end section 121a make with the Z direction are smaller than the angles that the lateral faces of the second end section 121b make with the Z direction, a micro-needle that is easy to insert into the surface of a living body and resists breaking at the position of the second end section 121b can be obtained. When such a structure is employed in which the angles that the lateral faces of the first end section 121a make with the Z direction are larger than the angles that the lateral faces of the second end section 121b make with the Z direction, a micro-needle that resists breaking over the entire length thereof can be obtained.

The micro-needle 121 shown in FIG. 5 further includes a middle section 121c interposed between the first end section 121a and the second end section 121b. The middle section 121c has a truncated quadrangular pyramid shape. The angles that the lateral faces of the middle section 121c make with the Z direction are larger than the angles that the lateral faces of the first end section 121amake with the Z direction and smaller than the angles that the lateral faces of the second end section 121b make with the Z direction.

As such, the micro-needle 121 may further includes the middle section 121c having a truncated cone or columnar shape different in the angles of inclinations of lateral faces from the first end section 121a and the second end section 121b. In the case where the angles of inclinations of lateral faces of the middle section 121c are between the angles of inclinations of lateral faces of the first end section 121a and the angles of inclinations of lateral faces of the second end section 121b, the physical properties of the micro-needle 121 can be gradually changed in the Z direction. When the structure shown in FIG. 5 is employed, the strength at and near the second end section 121b can be increased. Therefore, breaking of the micro-needle 121 at and near the second end section 121b can be suppressed.

The inclinations of the middle section 121c with respect to the Z direction may be smaller than the inclinations of the first end section 121a with respect to the Z direction and the inclinations of the second end section 121b with respect to the Z direction. For example, it is possible that the first end section 121a is a cone or truncated cone, the second end section 121b is a truncated cone, and the middle section 121c is a columnar. Such a structure is advantageous in suppressing breaking of the micro-needle 121 at and near the second end section 121b, and is useful when the tip of the micro-needle 121 must reach to a position far from the surface of a living body.

The micro-needle 121 shown in FIG. 6 has the structure in which it is tapered down from an end on the side of the support layer 11 to another end. The first end section 121a has a quadrangular pyramid shape. The second end section 121b has a quadrangular prism shape. As such, the micro-needle 121 whose second end section 121b has a columnar shape is useful when the tip of the micro-needle 121 must reach to a position far from the surface of a living body.

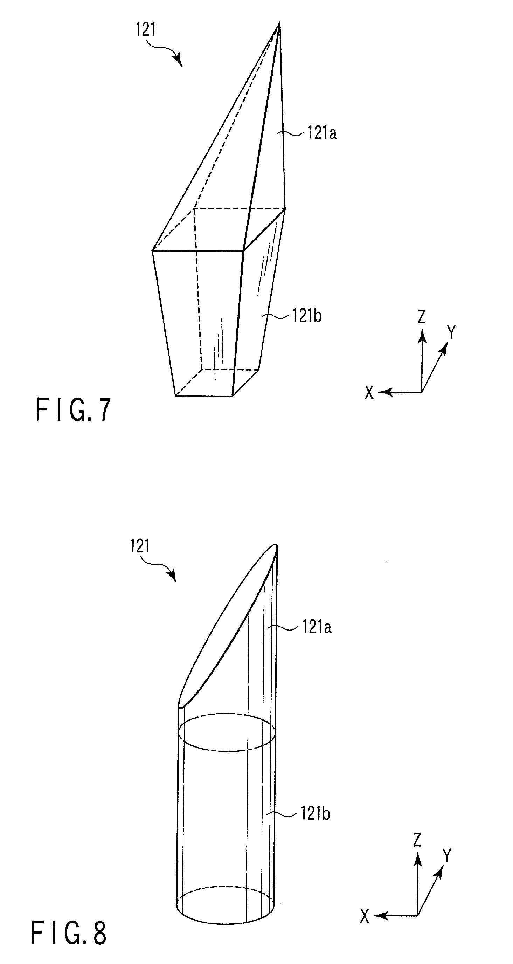

In the micro-needle 121 shown in FIG. 7, the first end section 121a has the structure in which it is tapered down from an end on the side of second end section 121b to another end. The second end section 121b has the structure in which it is tapered down from an end on the side of the first end section 121a to another end. To be more specific, the first end section 121a has an oblique quadrangular pyramid shape. The second end section 121b has a truncated quadrangular pyramid shape.

In the case where such a structure is employed, it is possible to make the inserted micro-needle 121 difficult to be withdrawn from the living body as compared with the case where the structure shown in FIG. 4 is employed. Further, in the case where such a structure is employed, it is possible to easily break the micro-needle 121 in the state that it is inserted into the living body as compared with the case where the structure shown in FIG. 4 is employed. Therefore, this structure is suitable for leaving the micro-needle 121 in the living body. When the micro-needle 121 contains a drug, a longer duration of the pharmacologic effect can be achieved by leaving the micro-needle 121 in the living body.

The micro-needle 121 shown in FIG. 8 has the structure in which it is tapered down from an end on the side of the support layer 11 to another end. The first end section 121a has a truncated circular cylinder shape. The second end section 121b has a circular cylinder shape. When the first end section 121a is a truncated cylinder as above, it is relatively easy to form a sharp tip.

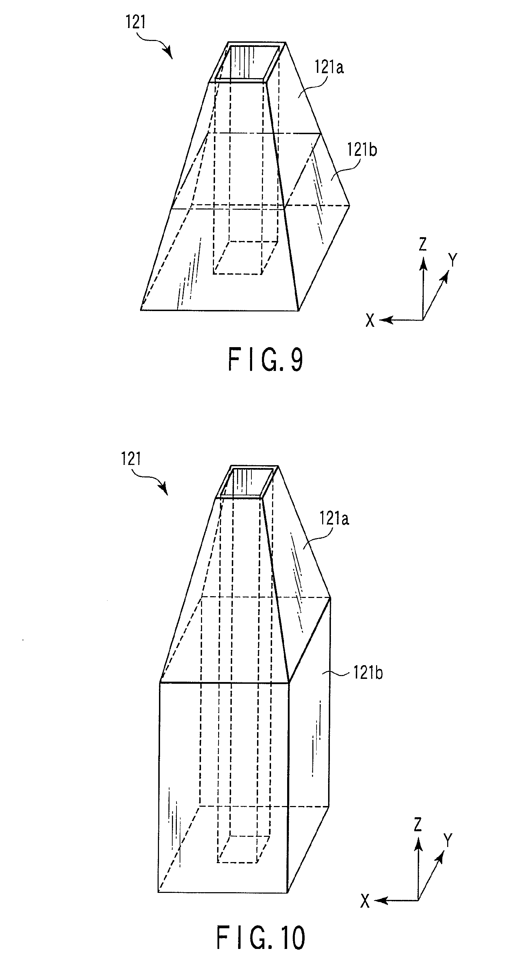

Each of the micro-needles 121 shown in FIGS. 9 and 10 has the structure in which it is tapered down from an end on the side of the support layer 11 to another end and is provided with a through-hole extending in the longitudinal direction. In each micro-needle 121, the first end section 121a has a truncated quadrangular pyramid shape provided with a through-hole extending in the height direction. In the micro-needle 121 shown in FIG. 9, the second end section 121b has a truncated quadrangular pyramid shape provided with a through-hole extending in the height direction. In the micro-needle 121 shown in FIG. 10, the second end section 121b has a quadrangular prism shape provided with a through-hole extending in the height direction.

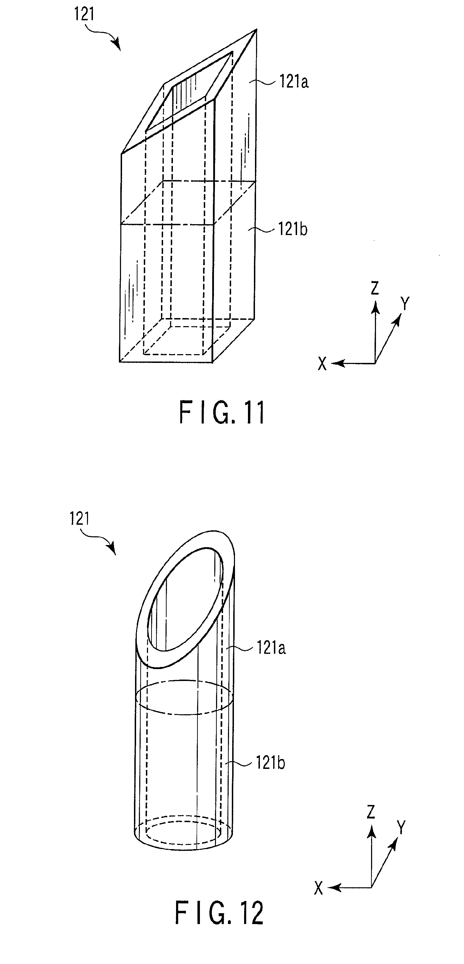

Each of the micro-needles 121 shown in FIGS. 11 and 12 has the structure in which it is tapered down from an end on the side of the support layer 11 to another end and is provided with a through-hole extending in the longitudinal direction. In the micro-needle 121 shown in FIG. 11, the first end section 121a has a truncated quadrangular prism shape provided with a through-hole extending in the height direction, while the second end section 121b has a right quadrangular prism shape provided with a through-hole extending in the height direction. In the micro-needle 121 shown in FIG. 12, the first end section 121a has a truncated circular cylinder shape provided with a through-hole extending in the height direction, while the second end section 121b has a right circular cylinder shape provided with a through-hole extending in the height direction.

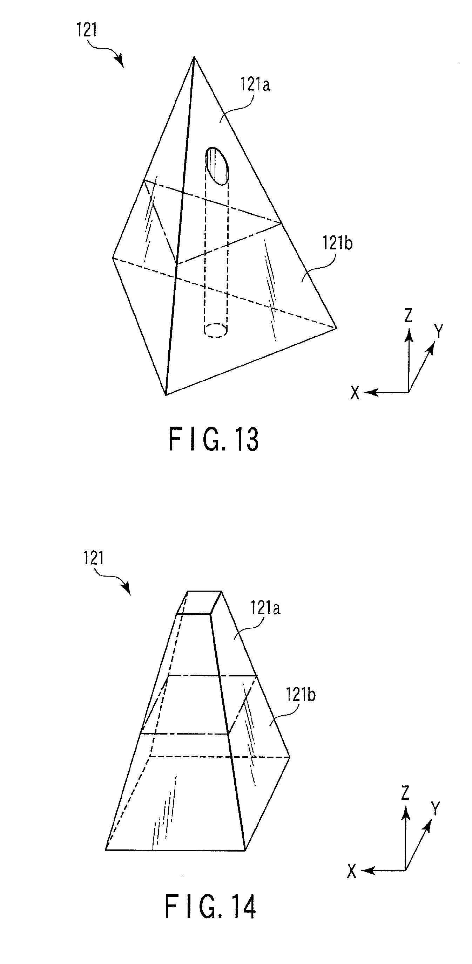

The micro-needle 121 shown in FIG. 13 has the structure in which it is tapered down from an end on the side of the support layer 11 to another end and is provided with a through-hole extending in the height direction. The first end section 121a has a triangular pyramid shape provided with a through-hole extending in the height direction. The second end section 121b has a truncated triangular pyramid shape provided with a through-hole extending in the height direction. In the micro-needle 121 shown in FIG. 13, one of the openings of the through-hole is located at the base of the triangular pyramid, while the other opening is located not at the vertex of the triangular pyramid but at the lateral face of the triangular pyramid.

When the micro-needle 121 is provided with a through-hole as shown in FIGS. 9 to 13, the through-hole can be filled with the feed substance such as the bioactive substance, for example. Thus, in this case, much more amount of the feed substance can be delivered into the living body as compared with the case where the through-hole is omitted.

Note that the micro-needle 121 may be provided with a recess instead of the through-hole. The recess can be filed with the feed substance such as the bioactive substance, for example. Thus, also in this case, much more amount of the feed substance can be delivered into the living body as compared with the case where the through-hole is omitted.

The through-hole formed in the micro-needle 121 can be used as a channel for transferring a substance out of the living body or into the living body. For example, in the case where blood collection or bloodletting is performed, the through-hoe can be used as a channel for transferring the blood out of or into the living body. Alternatively, a liquid substance can be delivered into the living body via the through-hole. When the through-hole is used for such a purpose, the support layer 11 may be provided with a channel that connects the through-hole with the exterior of the micro-needle patch 1.

Next, the "maximum apical angle" and the "minimum apical angle" will be described. Although the micro-needles 121 can employ various structures, in the present context, regardless of the structure of the micro-needles 121, the "maximum apical angle" and the "minimum apical angle" of the first end section 121a is defined as follows.

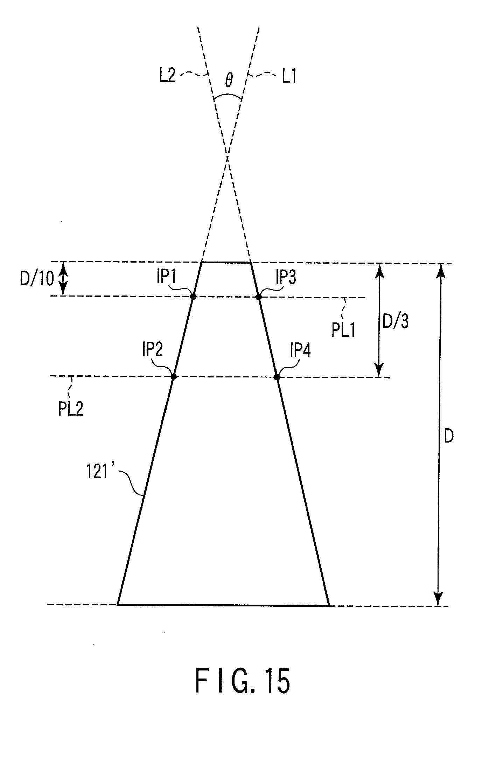

FIG. 14 is a perspective view schematically showing a still another example of modified micro-needle. FIG. 15 is a view showing an orthogonal projection of the micro-needle shown in FIG. 14 onto a plane perpendicular to the longitudinal direction thereof.

The micro-needle 121 shown in FIG. 14 has a structure in which it tapers down from an end on the side of the support layer 11 toward another end. To be more specific, the micro-needle 121 has a quadrangular pyramid shape in which all the cross sections perpendicular to the Z direction are square. FIG. 15 shows the orthogonal projection 121' of the micro-needle 121 on a plane parallel with the Z direction.

In FIG. 15, the reference symbol D denotes the dimension of the micro-needle 121 in the Z direction. The alternate long and short dash line PL1 denotes the plane that is perpendicular to the Z direction and spaced apart from the end of the micro-needle 121 on the side of the first end section 121a by a distance of D/10. The alternate long and short dash line PL2 denotes the plane that is perpendicular to the Z direction and spaced apart from the end of the micro-needle 121 on the side of the first end section 121a by a distance of D/3. The points IP1 and IP3 are intersection points of the contour of the orthogonal projection 121' with the plane PL1. The points IP2 and IP4 are intersection points of the contour of the orthogonal projection 121' with the plane PL2. The apical angle θ is the angle that the straight line L1 passing through the intersection points IP1 and IP2 forms with the straight line L2 passing through the intersection points IP3 and IP4.

In the case where the micro-needle 121 is a body of revolution having a symmetry axis parallel with the Z direction, the apical angle θ is not changed if the plane onto which the micro-needle 121 is projected is rotated about the axis parallel with the Z direction. By contrast, in the case where the micro-needle 121 does not have a symmetry axis parallel with the Z direction, the apical angle θ is changed when the plane onto which the micro-needle 121 is projected is rotated about the axis parallel with the Z direction. In any cases, the maximum apical angle and the minimum apical angle of the first end section 121a are the maximum value and the minimum value of the apical angle θ, respectively.

For example, in the case where the structure shown in FIG. 14 is employed, the maximum apical angle of the first end section 121a is the apical angle θ obtained when the micro-needle 121 is projected onto a plane perpendicular to the direction that forms angles of 45° with the X direction and the Y direction. Also in this case, the minimum apical angle of the first end section 121a is the apical angle θ obtained when the micro-needle 121 is projected onto a plane perpendicular to the X direction or the Y direction. Note that in the case where the micro-needle 121 has a symmetry axis parallel with the Z direction, the maximum apical angle is equal to the minimum apical angle.

The micro-needle patch 1 can be manufactured, for example, by the following method.

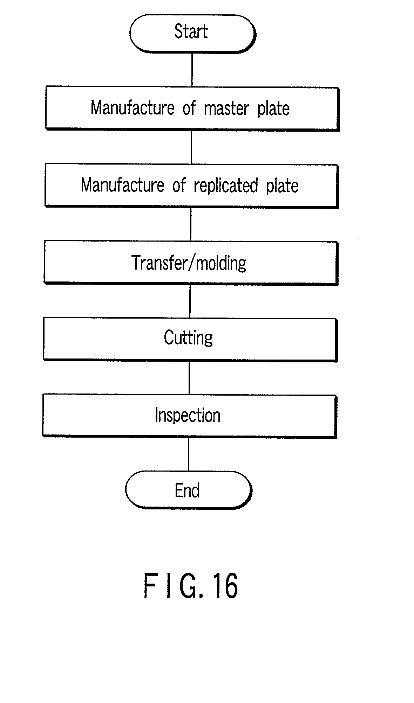

FIG. 16 is a flow-chart showing an example of a method for manufacturing a micro-needle patch.

According to this method, a master plate provide with protrusions is manufactured first. The protrusions are formed such that they have almost the same shapes and are arranged correspondingly with the micro-needles 121.

Next, using the master plate, a plate having recessed pattern corresponding to the protruding pattern is formed. Subsequently, using this plate, a replicated plate having a protruding pattern corresponding to the recessed pattern is formed.

Then, the replicated plate is pressed against a back surface of a film or sheet made of a raw material of the micro-needles 121, and the film or sheet is heated. To do so, the above-described protruding pattern is produced on a surface of the film or sheet. The film or sheet is removed from the replicated plate after cooled down sufficiently.

Next, the molded film or sheet is cut out into appropriate dimensions. Thus, the micro-needle patch 1 is obtained. Note that in ordinary cases, multiple micro-needle patches 1 are manufactured from a single film or sheet.

Then, the micro-needle patches 1 are subjected to an inspection. As above, the manufacture of the micro-needle patches 1 is completed.

In this method, the plate having the protruding pattern is used as a plate for forming a pattern on the film or sheet. Alternatively, as the plate for forming a pattern on the film or sheet, a plate having a recessed pattern or both of a plate having a protruding pattern and a plate having a recessed pattern may be used.

In the case where the feed substance is supported by the surface of the micro-needles 121, the above-described manufacturing process may further includes a step for spraying a fluid including the feed substance toward the micro-needle array 12, for example. In the case where a multilayered structure is employed in the support layer 11, the above-described process may further includes a step for adhering another layer on the film or sheet and/or a step for forming another layer on the film or sheet after the step for transferring the protruding pattern onto the film or sheet.

The film or sheet used in this method can be manufactured, for example, by the following method. Here, as an example, the method of manufacturing the film or sheet that can be used when the biocompatible material is chitin/chitosan will be described.

First, chitin is dissolved in a methanol solution of calcium compound. Next, a large amount of water is added to the solution so as to precipitate the chitin. Subsequently, calcium is removed from the precipitate by dialysis. Thus, a white gel having a chitin content of about 4 to 5% is obtained. Then, the gel is mixed with distilled water to prepare a suspension, and papermaking using this suspension is performed. Further, a laminar product is subjected to pressing and drying so as to obtain the film or sheet having a chitin content of 100%.

The micro-needle patch 1 can be manufactured by other methods. For example, the micro-needle array 12 may be formed using photolithography. In this case, a photomask that is provided with light-shielding portions corresponding to the micro-needles 121 can be used.

Next, examples of the present invention will be described.

### (Example 1)

In this example, micro-needle patches 1 each having the structure shown in FIG. 1 and differing in the structures of the micro-needles 121 from one another were manufactured. To be more specific, in each micro-needle patch 1, the number of the micro-needles 121 was 900, the micro-needles 121 had a truncated cone shape, and polylactic acid was used as the material thereof. Each micro-needle 121 having an apical angle of 12° or less was formed by drawing a part of a yarn made of polylactic acid and cutting it at the thinnest position thereof. Each micro-needle 121 having a wider apical angle was formed using a metal mold. The metal molds were formed using a micromachining technology.

Next, the maximum apical angle was measured for each of the micro-needle patches 1 by the method described with reference to FIGS. 14 and 15. To be more specific, a screen was placed parallel with the longitudinal direction of the micro-needles 121, and the micro-needles 121 were projected onto the screen using a 1x lens. Note that in this example, the maximum apical angle is equal to the minimum apical angle.

Then, for each of the micro-needle patches, the performances of the micro-needles were tested using a tension and compression-testing machine "TENSILON (trade mark)".

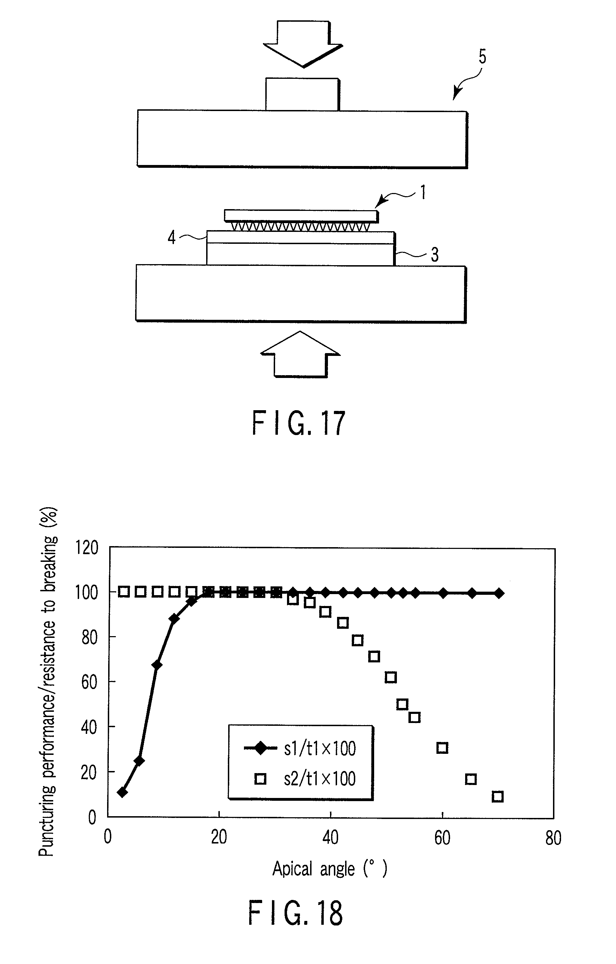

FIG. 17 is a view schematically showing a part of a tension and compression-testing machine. As shown in FIG. 17, a silicone rubber layer 3 and a micro-needle patch 1 were stacked with a skin of a rat 4 interposed therebetween, and the layered product was mounted on the tension and compression-testing machine 5. The skin of rat 4 was bought from CHARLES RIVEW JAPAN, INC. Then, a load of 5 kgf was applied to the layered product, and the proportion of the micro-needles 121 that could be inserted into the skin of rat and the proportion of the micro-needles 121 that were broken were obtained.

FIG. 18 is a graph showing relationships between an apical angle and a puncturing performance and a resistance to breaking of a micro-needle. In the figure, the abscissa denotes the maximum apical angle of the first end section 121a, while the ordinate denotes the puncturing performance and the resistance to breaking of the micro-needles 121. Note that in this figure, as the resistance to breaking of the micro-needles 121, plotted are the values s1/t1 × 100 each obtained by multiplying the ratio s1/t1 of the number s1 of the unbroken micro-needles 121 with respect to the total number t1 of the micro-needles 121 by 100. Note also that in this figure, as the puncturing performance, plotted are the values s2/s1 × 100 each obtained by multiplying the ratio s2/s1 of the number of the micro-needles 121 that could be inserted into the skin of rat with respect to the number s1 by 100.

As shown in FIG. 18, in the case where the apical angle was within a range of about 9° to about 53°, a puncturing performance of 50% or more and a resistance to breaking of 50% or more could be achieved. In the case where the apical angle was within a range of about 18° to about 30°, a puncturing performance over 90% and a resistance to breaking over 90% could be achieved. Further, in the case where the apical angle was within a range of about 28° to about 30°, a puncturing performance of almost 100% and a resistance to breaking of almost 100% could be achieved.

### (Example 2)

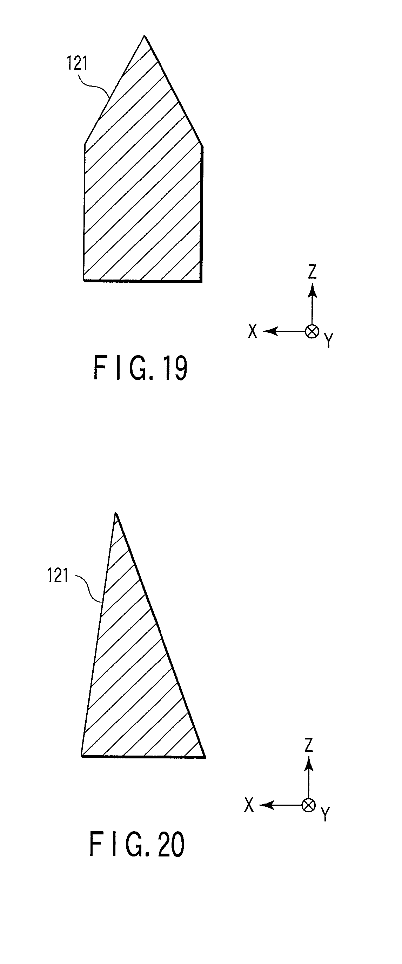

FIGS. 19 to 22 are sectional views schematically showing structures of micro-needles employed in Example 2. Each of the micro-needles shown in FIGS. 19 to 22 has a shape tapering down from an end toward another end and all the cross sections thereof perpendicular to the Z direction are circular.

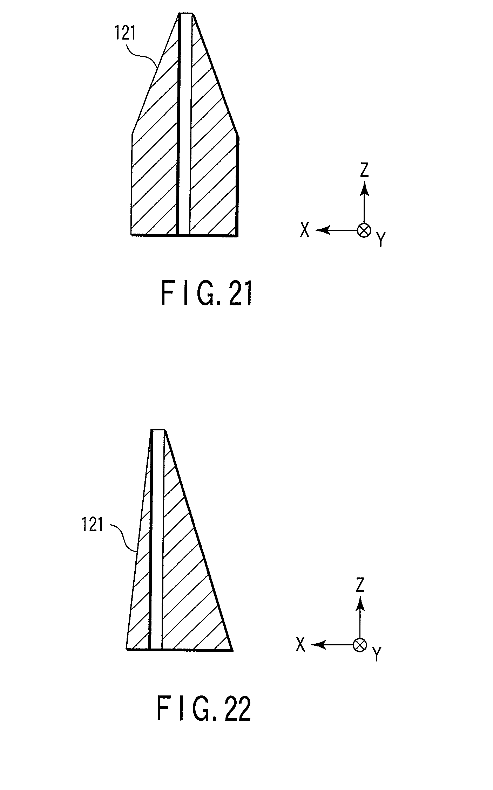

To be more specific, each of the micro-needles 121 shown in FIGS. 19 to 22 is a body of revolution having a symmetry axis parallel with the Z direction. The micro-needle 121 shown in FIG. 19 includes a first section 121a having a circular cone shape and a second section 121b having a circular cylinder shape. The micro-needle 121 shown in FIG. 21 is the same as the micro-needle 121 shown in FIG. 19 except that it is provided with a through-hole extending in a direction parallel with the Z direction.

Each of the micro-needles 121 shown in FIGS. 20 and 22 does not have a symmetry axis parallel with the Z direction. The micro-needle 121 shown in FIG. 22 has an oblique circular cone shape. The micro-needle 121 shown in FIG. 22 is the same as the micro-needle 121 shown in FIG. 20 except that it is provided with a through-hole extending in a direction parallel with the Z direction.

In this example, micro-needle patches 1 differing in the apical angles of the first end section 121a from one another were manufactured by the same method as that described in Example 1 except that the structures shown in FIGS. 19 to 22 were employed in the micro-needles 121. Note that the maximum dimension of the micro-needles 121 in the Z direction was about 300 µm. Note also that the diameter of the through-holes was about 20 µm.

The same tests as that described in Example 1 were performed on these micro-needles 121. As a result, the range of the apical angle within which an excellent puncturing performance and a high resistant to breaking were achieved was almost the same as that in Example 1.

### (Example 3)

In this example, micro-needle patches 1 differing in the structures of the micro-needles 121 from one another were manufactured by the same method as that described in Example 2 except that a copolymer of polylactic acid and glycolic acid was used instead of polylactic acid. Then, the same tests were performed on each of the micro-needle patches 1. As a result, the range of the apical angle within which an excellent puncturing performance and a high resistant to breaking were achieved was almost the same as that in Example 1.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A micro-needle comprising first and second end sections arranged in a longitudinal direction and including a biocompatible material, the first end section tapering down from an end of the first end section on a side of the second end section toward another end of the first end section, a minimum dimension of the first end section in a width direction perpendicular to the longitudinal direction being smaller than a minimum dimension of the second end section in the width direction, a maximum apical angle of the first end section falling within a range of 9 to 53°, the maximum apical angle being a maximum of apical angles each defined as an angle that a first straight line passing through first and second intersection points forms with a second straight line passing through third and fourth intersection points, the first and third intersection points being intersection points of a first plane and a contour of an orthogonal projection of the micro-needle on a projection plane parallel with the longitudinal direction, the second and fourth intersection points being intersection points of a second plane and the contour, the first plane being perpendicular to the longitudinal direction and spaced apart from the another end by one tenth of a length of the micro-needle in the longitudinal direction, and the second plane being perpendicular to the longitudinal direction and spaced apart from the another end by one third of the length.

2. The micro-needle according to claim 1, wherein a minimum of the apical angles falls within a range of 9 to 53°.

3. The micro-needle according to claim 1, wherein the maximum apical angle falls within a range of 20 to 30°.

4. The micro-needle according to claim 3, wherein the minimum apical angle falls within a range of 20 to 30°.

5. The micro-needle according to claim 1, wherein the micro-needle is tapered down from an end to another end.

6. The micro-needle according to claim 5, wherein the first end section has a cone shape and the second end section has a cylindrical shape.

7. The micro-needle according to claim 1, wherein the micro-needle has a cone shape.

8. The micro-needle according to claim 1, further includes a biologically active substance.

9. The micro-needle according to claim 1, wherein the biocompatible material includes polylactic acid.

10. The micro-needle according to claim 1, wherein the biocompatible material includes a copolymer of polylactic acid and glycolic acid.

11. The micro-needle according to claim 1, wherein the micro-needle is provided with a through-hole or recess extending in the longitudinal direction.

12. A micro-needle patch, comprising:
a support layer with first and second main surfaces; and
micro-needles each extending from the first main surface, each of the micro-needles being the micro-needle according to one of claims 1 to 10 supported by the first main surface at an end of the second end section.

13. The micro-needle patch according to claim 12, further comprising a biologically active substance supported by a surface of at least one of the first and second end sections.

14. The micro-needle patch according to claim 12, at least one of the micro-needles is provided with a through-hole or recess extending in the longitudinal direction.

15. The micro-needle according to claim 14, further comprising a functional material filling the through-hole or recess.
